(19) Europäisches Patentamt
European Patent Office
Office européen des brevets

(11) Veröffentlichungsnummer: **0 204 209**
**A2**

# (12) EUROPÄISCHE PATENTANMELDUNG

(21) Anmeldenummer: **86106845.0**

(22) Anmeldetag: **20.05.86**

(51) Int. Cl.⁴: **C07D 471/04**, C07D 471/10, A01N 43/90, //(C07D471/04,235:00,221:00)

(30) Priorität: **03.06.85 DE 3520260**

(43) Veröffentlichungstag der Anmeldung: **10.12.86 Patentblatt 86/50**

(84) Benannte Vertragsstaaten: **AT BE CH DE FR GB IT LI LU NL SE**

(71) Anmelder: **SCHERING AKTIENGESELLSCHAFT**
**Berlin und Bergkamen**
**Müllerstrasse 170/178 Postfach 65 03 11**
**D-1000 Berlin 65(DE)**

(72) Erfinder: **Franke, Wilfried, Dr.**
**Karl-Stieler-Strasse 2b**
**D-1000 Berlin 41(DE)**
Erfinder: **Nordhoff, Erhard, Dr.**
**Flughafenstrasse 18**
**D-1000 Berlin 44(DE)**
Erfinder: **Arndt, Friedrich, Dr.**
**Mühlenfeldstrasse 10**
**D-1000 Berlin 28(DE)**

## (54) Imidazo-Isochinolin-Trione, Verfahren zu ihrer Herstellung und diese Verbindungen enthaltende Mittel mit herbizider Wirkung.

(57) Es werden neue Imidazo [2,1-a] isochinolin-2,5,6(3H)-trione der allgemeinen Formel

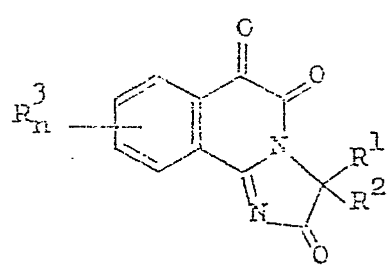

,

in der $R^1$, $R^2$, $R^3$ und n die in der Beschreibung genannten Bedeutungen haben, Verfahren zu ihrer Herstellung und diese Verbindungen enthaltende Mittel mit herbizider Wirkung beschrieben.

EP 0 204 209 A2

# IMIDAZO-ISOCHINOLIN-TRIONE, VERFAHREN ZU IHRER HERSTELLUNG UND DIESE VERBINDUNGEN ENTHALTENDE MITTEL MIT HERBIZIDER WIRKUNG

Die Erfindung betrifft neue Imidazo [2,1-a] isochinolin-2,5,6 (3H)-trione, Verfahren zu ihrer Herstellung und diese Verbindungen enthaltende Mittel mit herbizider Wirkung.

Die herbizide Wirkung von Imidazolinyl-Derivaten ist bereits bekannt (US-PS 4 188 487). Sie ist aber nicht immer ausreichend oder es treten Selektivitätsprobleme in landwirtschaftlich wichtigen Kulturen auf.

Aufgabe der vorliegenden Erfindung ist die Bereitstellung von neuen Verbindungen, die die Nachteile der bekannten Verbindungen nicht aufweisen und in ihren biologischen Eigenschaften diesen überlegen sind.

Diese Aufgabe wird erfindungsgemäß gelöst durch Imidazo [2,1-a] isochinolin-2,5,6(3H)-trione der allgemeinen Formel I

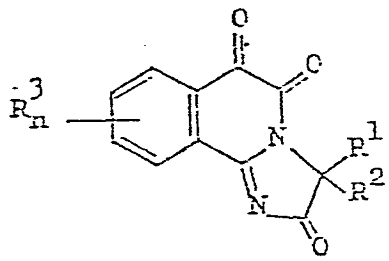

(I),

in der

$R^1$ einen gegebenenfalls ein-oder mehrfach durch Halogen substituierten $C_1$-$C_4$-Alkylrest,

$R^2$ einen gegebenenfalls ein-oder mehfach durch Halogen substituierten $C_1$-$C_4$-Alkyl-oder $C_3$-$C_6$-Cycloalkylrest oder

$R^1$ und $R^2$ gemeinsam mit dem benachbarten Kohlenstoffatom einen gegebenenfalls durch Methyl substituierten $C_2$-$C_5$-Alkylenrest,

$R^3$ gleich oder verschieden Wasserstoff, Halogen, einen gegebenenfalls ein-oder mehrfach durch Halogen substituierten $C_1$-$C_4$-Alkylrest, einen gegebenenfalls ein-oder mehrfach, gleich oder verschieden durch $C_1$-$C_6$-Alkyl, Halogen, $C_1$-$C_4$-Alkoxy, $C_1$-$C_4$-Alkylthio, Nitro und/oder Trifluormethyl substituierten Phenylrest oder eine der Gruppen $R^4X$, $R^5_2N$ oder $R^6CONH$,

$R^4$ einen gegebenenfalls ein-oder mehrfach durch Halogen, Phenoxy und/oder $C_1$-$C_4$-Alkoxy substituierten $C_1$-$C_{12}$-Alkyl-, $C_2$-$C_{12}$-Alkenyl-oder $C_2$-$C_{12}$-Alkinylrest, einen gegebenenfalls ein-oder mehrfach durch Sauerstoff unterbrochenen $C_2$-$C_8$-Alkylrest, einen gegebenenfalls ein-oder mehrfach durch Halogen, $C_1$-$C_4$-Alkyl und/oder $C_1$-$C_4$-Alkoxy substituierten $C_3$-$C_6$-Cycloalkylrest, oder einen gegebenenfalls ein-oder mehrfach, gleich oder verschieden durch $C_1$-$C_6$-Alkyl, Halogen, $C_1$-$C_4$-Alkoxy, $C_1$-$C_4$-Alkylthio, Nitro und/oder Trifluormethyl substituierten Phenyl-oder Phenyl-$C_1$-$C_4$-Alkylrest,

X O oder S,

$R^5$ einen gegebenenfalls ein-oder mehrfach durch Halogen substituierten $C_1$-$C_4$-Alkylrest,

$R^6$ einen gegebenenfalls ein-oder mehrfach durch Halogen substituierten $C_1$-$C_{12}$-Alkylrest oder einen gegebenenfalls ein-oder mehrfach, gleich oder verschieden durch $C_1$-$C_6$-Alkyl, Halogen, $C_1$-$C_4$-Alkoxy, Nitro und/oder Trifluormethyl substituierten Phenylrest und

n 1, 2, 3 oder 4

bedeuten.

-Von den erfindungsgemäßen Verbindungen zeichnen sich durch ihre herbizide Wirkung insbesondere diejenigen aus, bei denen in der allgemeinen Formel I $R^1$ Methyl, $R^2$ Methyl, Ethyl, Isopropyl und Cyclopropyl, $R^3$ Wasserstoff, $C_1$-$C_4$-Alkoxy, Allyloxy, Benzyloxy, Phenyloxy, und n 1 bedeuten.

Bei der Anwendung dieser Wirkstoffe als herbizide Mittel zeigen diese im Vergleich zu existierenden Standards eine überlegene Wirksamkeit.

Die erfindungsgemäßen Verbindungen lassen sich zum Beispiel herstellen, indem man

a) Amide der allgemeinen Formel II

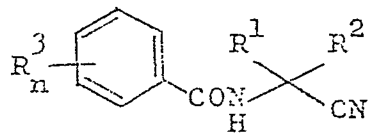

(II)

mit Oxalylchlorid umsetzt, oder

b) Imidazolinone der allgemeinen Formel III

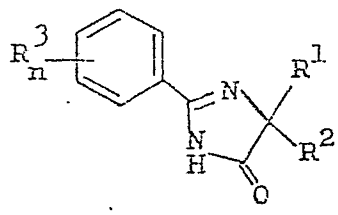

(III)

gegebenenfalls in Form der Hydrochloride mit Oxalylchlorid umsetzt, oder

c) Imidazolinone der allgemeinen Formel III

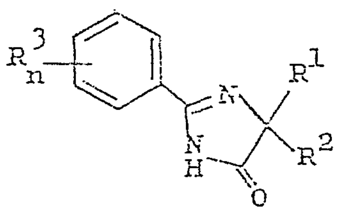

(III)

zunächst mit Alkyllithiumverbindungen der allgemeinen Formel IV

$R^7$ -Li (IV)

metalliert und anschließend mit Oxalsäureestern der allgemeinen Formel V

$R^8O_2C$ -$CO_2R^8$ (V)

umsetzt, wobei $R^1$, $R^2$, $R^3$ und n die oben angegebene Bedeutung haben und $R^7$ und $R^8$ jeweils für $C_1$-$C_4$-Alkyl steht.

Die Ausgangsstoffe zur Herstellung der erfindungsgemäßen Verbindungen sind bekannt oder können nach an sich bekannten Verfahren hergestellt werden.

Die Umsetzung der Reaktionspartner nach den erfindungsgemäßen Verfahren a) oder b) erfolgt zwischen 0° und 170°C, im allgemeinen jedoch zwischen Raumtemperatur und Rückflußtemperatur des entsprechenden Reaktionsgemisches.

Die Reaktionsdauer beträgt etwa 0,5 bis 48 Stunden. Das Oxalylchlorid wird in einer Menge von 1 bis 5 Moläquivalenten, bezogen auf das Ausgangsmaterial, eingesetzt.

Die Umsetzung der Reaktionspartner erfolgt ohne oder unter Verwendung eines geeigneten inerten Lösungsmittels. Als Lösungsmittel seien beispielsweise genannt Halogenkohlenwasserstoffe, wie Methylenchlorid,Dichlorethan oder Trichlorethylen, aromatische gegebenenfalls halogenierte Kohlenwasserstoffe, wie Benzol, Toluol, Xylol, Chlorbenzol, Dichlorbenzol oder Brombenzol, oder andere gegenüber den Reaktionspartnern inerte Lösungsmittel, wie Nitromethan, Nitroethan oder Schwefelkohlenstoff.

Gegebenenfalls können dem Reaktionsgemisch auch übliche Friedel-Crafts-Katalysatoren, wie zum Beispiel $AlCl_3$, Dimethylaluminiumchlorid, $SnCl_4$ oder Bortrifluorid-Etherat-Komplex zugesetzt werden.

Die Umsetzung der Reaktionspartner nach dem erfindungsgemäßen Verfahren c) erfolgt zwischen -78 und +70°C, im allgemeinen jedoch zwischen -78°C und Raumtemperatur.

Die Reaktionsdauer beträgt etwa 0,5 bis 48 Stunden.

Die Alkyllithiumverbindung der allgemeinen Formel IV wird in einer Menge von 2 bis 3 Moläquivalenten, bezogen auf das Ausgangsmaterial eingesetzt; der Oxalsäureester der allgemeinen Formel V in einer Menge von 1 bis 2 Moläquivalenten. Die Umsetzung der Reaktionspartner erfolgt unter Verwendung aprotischer Lösungsmittel, vorzugsweise Tetrahydrofuran.

Gegebenenfalls kann auch unter Zusatz von Tetramethylethylendiamin oder Hexamethylphosphorsäuretriamid gearbeitet werden.

Die so hergestellten Verbindungen werden nach üblichen Verfahren aus dem Reaktionsgemisch isoliert, beispielsweise durch Fällung, durch Destillation bei normalem oder vermindertem Druck, und die Rohprodukte, die gegebenenfalls aus Isomerengemischen bestehen können, durch Umkristallisation oder Säulenchromatographie gereinigt beziehungsweise getrennt.

Die erfindungsgemäßen Verbindungen stellen in der Regel gelblich gefärbte, geruchlose, kristalline oder zähflüssige Substanzen dar, die zum Teil schwerlöslich in Wasser und Alkoholen, wie Methanol, mäßig bis gut löslich in halogenierten Kohlenwasserstoffen, wie Chloroform, Sulfoxiden, wie Dimethylsulfoxid, Estern, wie Essigester, und Ethern, wie Diethylether oder Diisopropylether, sind.

Die erfindungsgemäßen Mittel zeigen in besonders hervorzuhebender Weise sowohl eine große Breitenwirkung bei niedrigen Aufwandmengen gegen schwer bekämpfbare monokotyle sowie dikotyle, annuelle und perennierende Unkräuter als auch eine hohe Selektivität in Hauptkulturen bei Anwendung im Vor-und Nachauflauf-Verfahren.

Von besonderem Vorteil ist, daß die Verbindungen in Aufwandmengen von etwa 0,05 kg Wirkstoff/ha das Aufkommen von einkeim-und zweikeimblättrigen Unkräutern in Nutzpflanzenkulturen hemmen oder sogar verhindern, ohne die Kulturen bis zu Aufwandmengen von etwa 3 kg Wirkstoff/ha zu schädigen.

Die Verbindugnen werden durch Wurzeln und Blätter aufgenommen und in der Pflanze verteilt unter Anreicherung in meristematischen Regionen. In behandelten Pflanzen wird das Wachstum bald nach der Spritzung unterbunden, und Chlorose tritt auf. In perennierenden Unkräutern werden die Verbindungen in unterirdische Teile transportiert, so daß der Wiederaustrieb verhindert wird.

Unkrautgattungen, die bekämpft werden, sind zum Beispiel Viola, Stellaria, Abutilon, Matricaria, Chrysanthemum, Ipomoea, Solanum, Brassica, Helianthus, Avena, Alopecurus, Echinochloa, Setaria, Cyperus, Mentha und andere.

Die Anwendung kann in Hauptkulturen wie Weizen, Roggen, Gerste, Reis und Plantagenkulturen erfolgen.

Die Aufwandmengen für die herbizide Wirkung betragen je nach Anwendungsziel im allgemeinen 0,05 -3 kg Wirkstoff/ha, es können jedoch gegebenenfalls auch höhere Aufwandmengen eingesetzt werden. Mit höheren Aufwandmengen ist auch eine totale Unkrautbekämpfung möglich. Je nach Ziel kann die Anwendung im Vor-oder Nachauflauf erfolgen.

Die erfindungsgemäßen Verbindungen können entweder allein, in Mischung miteinander oder mit anderen Wirkstoffen angewendet werden. Gegebenenfalls können andere Pflanzenschutz-oder Schädlingsbekämpfungsmittel je nach dem gewünschten Zweck zugesetzt werden.

Sofern eine Verbreiterung des Wirkungsspektrums beabsichtigt ist, können auch andere Herbizide zugesetzt werden. Beispielsweise eignen sich als herbizid wirksame Mischungspartner diejenigen Wirkstoffe, die in Weed Abstracts, Vol. 34, No. 3, 1985, unter dem Titel "List of common names and abbreviations employed for currently used herbicides and plant growth regulators in Weed Abstracts" aufgeführt sind.

Eine Förderung der Wirkintensität und der Wirkungsgeschwindigkeit kann zum Beispiel durch wirkungssteigernde Zusätze, wie organische Lösungsmittel, Netzmittel und Öle, erzielt werden. Solche Zusätze lassen daher gegebenenfalls eine Verringerung der Wirkstoffdosierung zu.

Als Mischungspartner können außerdem Phospholipide verwendet werden, zum Beispiel solche aus der Gruppe Phosphatidylcholin, den hydrierten Phosphatidylcholinen, Phosphatidylethanolamin, den N-Acyl-phosphatidylethanolaminen, Phosphatidylinosit, Phosphatidylserin, Lysolecithin und Phosphatidylglycerol.

Zweckmäßig werden die gekennzeichneten Wirkstoffe oder deren Mischungen in Form von Zubereitungen wie Pulvern, Streumitteln, Granulaten, Lösungen, Emulsionen oder Suspensionen, unter Zusatz von flüssigen und/oder festen Trägerstoffen beziehungsweise Verdünnungsmitteln und gegebenenfalls Haft-, Netz-, Emulgier-und/oder Dispergierhilfsmitteln angewandt.

Geeignete flüssige Trägerstoff sind zum Beispiel aliphatische und aromatische Kohlenwasserstoffe wie Benzol, Toluol, Xylol, Cyclohexanon, Isophoron, Dimethylsulfoxid, Dimethylformamid, weiterhin Mineralölfraktionen und Pflanzenöle.

Als feste Trägerstoffe eignen sich Mineralien, zum Beispiel Bentonit, Silicagel, Talkum, Kaolin, Attapulgit, Kalkstein, und pflanzliche Produkte, zum Beispiel Mehle.

An oberflächenaktiven Stoffen sind zu nennen zum Beispiel Calciumligninsulfonat, Polyethylenalkylphenylether, Naphthalinsulfonsäuren und deren Salze, Phenolsulfonsäuren und deren Salze, Formaldehydkondensate, Fettalkoholsulfate sowie substituierte Benzolsulfonsäuren und deren Salze.

Der Anteil des bzw. der Wirkstoffe(s) in den verschiedenen Zubereitungen kann in weiten Grenzen variieren. Beispielsweise enthalten die Mittel etwa 10 bis 90 Gewichtsprozent Wirkstoff, etwa 90 bis 10 Gewichtsprozent flüssige oder feste Trägerstoffe sowie gegebenenfalls bis zu 20 Gewichtsprozent oberflächenaktive Stoffe.

Die Ausbringung der Mittel kann in üblicher Weise erfolgen, zum Beispiel mit Wasser als Träger in Spritzbrühmengen etwa 100 bis 1000 Liter/ha. Eine Anwendung der Mittel im sogenannten Low-Volume und Ultra-Low-Volume-Verfahren ist ebenso möglich wie ihre Applikation in Form von sogenannten Mikrogranulaten.

Die Herstellung dieser Zubereitungen kann in an sich bekannter Art und Weise, zum Beispiel durch Mahl-oder Mischverfahren, durchgeführt werden. Gewünschtenfalls können Zubereitungen der Einzelkomponenten auch erst kurz vor ihrer Verwendung gemischt werden, wie es zum Beispiel im sogenannten Tankmixverfahren in der Praxis durchgeführt wird.

Zur Herstellung der verschiedenen Zubereitungen werden zum Beispiel die folgende Bestandteile eingesetzt:

A. ) Spritzpulver

1. ) 20 Gewichtsprozent Wirkstoff

10 Gewichtsprozent Calciumsalz der Ligninsulfonsäure

68 Gewichtsprozent Kaolin

2 Gewichtsprozent Dialkylnaphthalinsulfonat

2. ) 20 Gewichtsprozent Wirkstoff

35 Gewichtsprozent Bentonit

35 Gewichtsprozent kolloidale Kieselsäure

8 Gewichtsprozent Calciumsalz der Ligninsulfonsäure

2 Gewichtsprozent Natriumsalz des N-Methyl-N-oleyl-taurins

3. ) 40 Gewichtsprozent Wirkstoff

25 Gewichtsprozent Kaolin

25 Gewichtsprozent kolloidale Kieselsäure

8 Gewichtsprozent Calciumsalz der Ligninsulfonsäure

2 Gewichtsprozent Dialkylnaphthalinsulfonat

4. ) 20 Gewichtsprozent Wirkstoff

8 Gewichtsprozent Calciumsalz der Ligninsulfonsäure

2 Gewichtsprozent Natriumsalz des N-Methyl-N-oleyl-taurins

70 Gewichtsprozent Kreide

B. ) Emulsionskonzentrat

10 Gewichtsprozent Wirkstoff

80 Gewichtsprozent Isophoron

10 Gewichtsprozent polyethoxyliertes Sorbitanpalmitat

C. ) Staub-Formulierung

40 Gewichtsprozent Wirkstoff

57 Gewichtsprozent Talkum

3 Gewichtsprozent Natriumsalz des N-Methyl-N-oleyl-taurins

Die nachfolgenden Beispiele erläutern die Herstellung der erfindungsgemäßen Verbindungen:

Beispiel 1

3-Isopropyl-9-methoxy-3-methyl-imidazo [2,1-a] isochinolin-2,5,6(3H)-trion

Zu einer Lösung aus 35,5 g (0,28 mol) Oxalsäuredichlorid in 300 ml Orthodichlorbenzol tropft man bei Raumtemperatur eine Lösung von 14,8 g (0,06 mol) N-(1-Cyan-1,2-dimethylpropyl)-3-methoxybenzoesäureamid in 300 ml Orthodichlorbenzol und rührt anschließend 3 Stunden bei 120°C. Nach dem Abkühlen wird die Reaktionslösung mit 1000 ml 0,3 molarer $K_2CO_3$-Lösung versetzt und nach Phasentrennung die organische Phase zur Trockne eingeengt. Der Rückstand wird mit Methanol zur Kristallisation gebracht.

Ausbeute: 5,2 g = 29 % der Theorie

Fp.: 231°C

Das Ausgangsmaterial N-(1-Cyan-1,2-dimethylpropyl)-3-methoxy-benzoesäureamid wurde wie folgt hergestellt:

56 g (0,33 mol) 3-Methoxybenzoesäurechlorid in 50 ml Methylenchlorid werden unter Kühlung zur eine Lösung aus 43,3 g 2-Amino-2,3-dimethylbutyronitril (85 %ig, 0,33 mol) und 95 ml Triäthylamin in 100 ml Methylenchlorid getropft, über Nacht stehengelassen, auf Wasser gegeben und mit Methylenchlorid extrahiert. Die organischen Phasen werden mit Wasser chloridfrei gewaschen, getrocknet und eingeengt. Das Rohprodukt wird aus Diisopropylether umkristallisiert.

Ausbeute: 70 g = 86 % der Theorie

Fp.: 66°C

Analog dem oben genannten Verfahren wurden die folgenden Benzoesäureamide hergestellt:

N-(1-Cyan-1-methylethyl)-3-methoxybenzoesäureamid (Fp. 81° C)

N-(1-Cyan-1-methylpropyl)-3-methoxybenzoesäureamid (Öl)

N-(1-Cyan-1-methylbutyl)-3-methoxybenzoesäureamid (Öl)

N-(1-Cyan-1,2-dimethylpropyl)-3-propoxybenzoesäureamid (Öl)

N-(1-Cyan-1,2-dimethylpropyl)-3-ethoxybenzoesäureamid (Öl)

N-(1-Cyan-1,2-dimethylpropyl)-3-isopropoxybenzoesäureamid (Öl).

Beispiel 2

3-Isopropyl-9-isopropyloxy-3-methyl-imidazo [2,1-a] -isochinolin-2,5,6(3H)-trion

Eine Lösung aus 1 g (3,6 mmol) 4,5-Dihydro-4-isopropyl-2-(3-isopropyloxyphenyl)-4-methyl-5-oxo-1H-imidazol und 1,9 g (15 mmol) Oxalsäuredichlorid in 80 ml Orthodichlorbenzol wird 8 Stunden bei 120° C gerührt. Nach dem Abkühlen wird mit 0,2 molarer wäßriger $K_2CO_3$-Lösung neutralisiert, die organische Phase abgetrennt und zur Trockne eingeengt. Der Rückstand wird mit Diisopropylether zur Kristallisation gebracht.

Ausbeute: 0,65 g = 55 % der Theorie

Fp.: 133° C

Das Ausgangsmaterial 4,5-Dihydro-4-isopropyl-2-(3-methoxyphenyl)-4-methyl-5-oxo-1H-imidazol wurde wie folgt hergestellt:

Eine Lösung aus 26,4 g (0,1 mol) N-(1-Aminocarbonyl-1,2-dimethylpropyl)-3-methoxybenzoesäureamid und 20,8 g (0,1 mol) $PCl_5$ in 80 ml $POCl_3$ wird 12 Stunden bei Raumtemperatur gerührt. Danach kühlt man auf 5-10° C ab und filtriert das erhaltene Hydrochlorid des gewünschten Produkts ab.

Ausbeute: 13 g Hydrochlorid = 55 % der Theorie

Fp.: 210 -215° C

Die $POCl_3$-Lösung wird langsam auf Eiswasser gegeben und unter Kühlung mit gesättigter N $a_2CO_3$-Lösung auf pH 8 eingestellt. Nach der Extraktion mit Methylenchlorid wird die organische Phase getrocknet, eingeengt und das Rohprodukt aus Hexan/Diethylether umkristallisiert.

Ausbeute: 3 g = 14,5 % der Theorie

Fp.: 132° C

Beispiel 3

3-Isopropyl-3-methyl-imidazo [2,1-a] isochinolin-2,5,6(3H)-trion

Zu einer Lösung aus 4,32 g (20 mmol) 4,5-Dihydro-4-isopropyl-4-methyl-5-oxo-2-phenyl-1H-imidazol in 40 ml Tetrahydrofuran werden unter $N_2$-Atmosphäre bei -65 bis -72°C 28 ml sec.-Butyllithium (12%ige Lösung in Cyclohexan) getropft. Nach 1,5 Stunden Rühren bei -35 bis -40°C tropft man 3,2 g (22 mmol) Oxalsäurediethylester, gelöst in 25 ml Tetrahydrofuran, zu und läßt innerhalb von 12 Stunden auf Raumtemperatur kommen. Die Reaktionslösung wird auf Eiswasser gegeben, mit verdünnter Schwefelsäure vorsichtig angesäuert, die organische Phase abgetrennt und die wäßrige Phase 2 mal mit Methylenchlorid extrahiert. Nach dem Trocknen über Magnesiumsulfat und Entfernen des Lösungsmittels wird der Rückstand zunächst aus Diisopropylether/Isopropanol und dann aus Ethanol umkristallisiert.

Ausbeute: 1,46 g = 27 % der Theorie

Fp.: 245° C

In analoger Weise wurden auch die folgenden Verbindungen hergestellt:

| Beispiel Nr. | $R^1$ | $R^2$ | $R^3$ | Fp(°C) |
|---|---|---|---|---|
| 4 | $-CH_3$ | $-CH(CH_3)_2$ | $7-OCH_3$; $9-OCH_3$ | 208 |
| 5 | $-CH_3$ | $-CH(CH_3)_2$ | $9-OCH_2CH_3$ | 197 |
| 6 | $-CH_3$ | $-CH(CH_3)_2$ | $9-O(CH_2)_2CH_3$ | 153 |
| 7 | $-CH_3$ | $-CH(CH_3)_2$ | $7-OCH(CH_3)_2$ | 142 |
| 8 | $-CH_3$ | $-(CH_2)_2CH_3$ | $9-OCH_3$ | 116 |
| 9 | $-CH_3$ | $-CH_2CH_3$ | $9-OCH_3$ | 187 |
| 10 | $-CH_3$ | $-CH(CH_3)_2$ | $9-OCH_2CH=CH_2$ | 141 |
| 11 | $-CH_3$ | $-CH(CH_3)_2$ | $7-OCH_2CH=CH_2$ | 152 |
| 12 | $-CH_3$ | $-CH(CH_3)_2$ | $9-O(CH_2)_5CH_3$ | 117 |
| 13 | $-CH_3$ | $-CH(CH_3)_2$ | $9-OC_6H_5$ | 155 |
| 14 | $-CH_3$ | $-CH(CH_3)_2$ | $9-OCH_2C_6H_5$ | 170 |
| 15 | $-CH_3$ | $-CH(CH_3)_2$ | $9-O(CH_2)_2OC_6H_5$ | 136 |
| 16 | $-CH_3$ | $-CH(CH_3)_2$ | $8-OCH_3$; $9-OCH_3$ | > 220 |
| 17 | $-CH_3$ | $-CH_3$ | $9-OCH_3$ | >225 |
| 18 | $-CH_3$ | $-CH(CH_3)_2$ | $9-O(CH_2)_{11}CH_3$ | 82 |
| 19 | $-CH_3$ | $-CH(CH_3)_2$ | $9-OCH_2CHBrCH_2Br$ | 158 |
| 20 | $-CH_3$ | 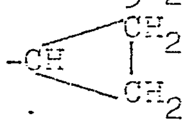 | $9-OCH_2CH_3$ | 204 |
| 21 | $-CH_3$ | 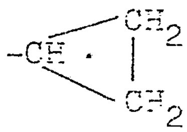 | $9-OCH_3$ | 186 |
| 22 | $-CH_3$ | $-CH(CH_3)_2$ | $9-O(CH_2)_2CH(CH_3)_2$ | 139 |
| 23 | $-CH_3$ | $-CH(CH_3)_2$ | $9-OCH(CH_3)CH_2CH_3$ | 158 |
| 24 | $-CH_3$ | $-CH(CH_3)_2$ | $9-OCH(CH_3)(CH_2)_2CH_3$ | 150 |

Die folgenden Beispiele dienen zur Erläuterung der Anwendungsmöglichkeiten der erfindungsgemäßen Verbindungen, die in Form ihrer Zubereitungen erfolgte:

Beispiel A

Im Gewächshaus wurden die in der Tabelle aufgeführten erfindungsgemäßen Verbindungen in einer Aufwandmenge von 3 kg Wirkstoff/ha emulgiert in 500 Liter Wasser/ha auf Brassica spp. und Solanum spp. als Testpflanzen im Vor-und Nachauflauf-Verfahren gespritzt. Drei Wochen nach der Behandlung wurde das Behandlungsergebnis bonitiert, wobei folgendes Schema verwendet wurde:

0 = keine Wirkung

1 = geringe Wirkung oder schwache Inhibition des Pflanzenwuchses

2 = mittlere Wirkung oder Inhibition des Pflanzenwuchses

3 = totale Wuchsinhibition

4 = totale Vernichtung der Pflanzen

| Erfindungsgemäße Verbindungen | Vorauflauf Brassica spp. | Vorauflauf Solanum spp. | Nachauflauf Brassica spp. | Nachauflauf Solanum spp. |
|---|---|---|---|---|
| Beispiel 1 | 3 | 3 | 4 | 3 |
| Beispiel 3 | | | 4 | 4 |
| Beispiel 9 | | | 3 | 3 |
| Beispiel 10 | | | 3 | 3 |
| Beispiel 13 | | | 2 | 2 |
| Beispiel 14 | | | 2 | 2 |
| Beispiel 16 | | | 2 | 2 |
| Beispiel 17 | | | 2 | 2 |
| Beispiel 19 | | | 2 | 2 |
| Beispiel 20 | | | 3 | 3 |
| Beispiel 21 | | | 4 | 4 |
| Beispiel 22 | | | 3 | 3 |
| Beispiel 23 | | | 3 | 3 |
| Beispiel 24 | | | 3 | 3 |

Beispiel B

Im Gewächshaus wurden die aufgeführten Pflanzen nach dem Auflaufen mit den aufgeführten Verbindungen in einer Aufwandmenge von 300 g Wirkstoff/ha behandet. Die Verbindungen wurden zu diesem Zweck als acetonige Lösung mit 500 Liter Wasser/ha gleichmäßig über die Pflanzen versprüht. Drei Wochen nach der Behandlung zeigten die erfindungsgemäßen Verbindungen eine hohe Selektivität in Weizen bei ausgezeichneter Wirkung gegen das Unkraut. Das Vergleichsmittel war deutlich weniger wirksam.

Die Bonitur erfolgte nach folgendem Schema:

0 = keine Wirkung

1 = geringe Wirkung oder schwache Inhibition des Pflanzenwuchses

2 = mittlere Wirkung oder Inhibition des Pflanzenwuchses

3 = totale Wuchsinhibition

4 = totale Vernichtung der Pflanzen

| Erfindungsgemäße Verbindungen | Triticum aestivum | Ipomoea spp. | Solanum spp. | Brassica napus | Helianthus annuus | Abutilon spp. | Viola spp. | Setaria spp. | Echinochloa crus-galli |
|---|---|---|---|---|---|---|---|---|---|
| Beispiel 1 | 0 | 3 | 3 | 3 | 3 | 3 | 3 | 3 | 3 |
| Beispiel 3 | 0 | 3 | 3 | 3 | 3 | 3 | 3 | 3 | 3 |
| **Vergleichsmittel** | | | | | | | | | |
| 2-(5-Isopropyl-5-methyl-4-oxo-2-imidazolin-2-yl)-4(5)-methyl-benzoesäure-methylester | 0 | 0 | 1 | 1 | 0 | 1 | 1 | 0 | 0 |

**Ansprüche**

1. Imidazo [2,1-a] isochinolin-2,5,6(3H)-trione der allgemeinen Formel I

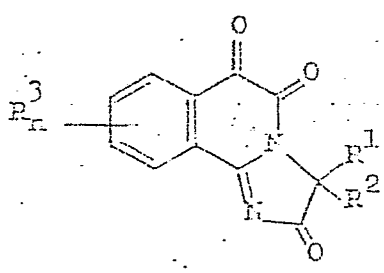

(I),

in der

$R^1$ einen gegebenenfalls ein-oder mehrfach durch Halogen substituierten $C_1$-$C_4$-Alkylrest,

$R^2$ einen gegebenenfalls ein-oder mehrfach durch Halogen substituierten $C_1$-$C_4$-Alkyl-oder $C_3$-$C_6$-Cycloalkylrest oder

$R^1$ und $R^2$ gemeinsam mit dem benachbarten Kohlenstoffatom einen gegebenenfalls durch Methyl substituierten $C_2$-$C_5$-Alkylenrest,

$R^3$ gleich oder verschieden Wasserstoff, Halogen, einen gegebenenfalls ein-oder mehrfach durch Halogen substituierten $C_1$-$C_4$-Alkylrest, einen gegebenenfalls ein-oder mehrfach, gleich oder verschieden durch $C_1$-$C_6$-Alkyl, Halogen, $C_1$-$C_4$-Alkoxy, $C_1$-$C_4$-Alkylthio, Nitro und/oder Trifluormethyl substituierten Phenylrest oder der Gruppen $R^4X$, $R^5_2N$ oder $R^6CONH$,

$R^4$ einen gegebenenfalls ein-oder mehrfach durch Halogen, Phenoxy und/oder $C_1$-$C_4$-Alkoxy substituierten $C_1$-$C_{12}$-Alkyl-, $C_2$-$C_{12}$-Alkenyl-oder $C_2$-$C_{12}$-Alkinylrest, einen gegebenenfalls ein-oder mehrfach durch Sauerstoff unterbrochenen $C_2$-$C_8$-Alkylrest, einen gegebenenfalls ein-oder mehrfach durch Halogen, $C_1$-$C_4$-Alkyl und/oder $C_1$-$C_4$-Alkoxy substituierten $C_3$-$C_6$-Cycloalkylrest, oder einen gegebenenfalls ein-oder

mehrfach, gleich oder verschieden durch $C_1$-$C_6$-Alkyl, Halogen, $C_1$-$C_4$-Alkoxy, $C_1$-$C_4$-Alkylthio, Nitro und/oder Trifluormethyl substituierten Phenyl-oder Phenyl-$C_1$-$C_4$-Alkylrest,

X O oder S,

$R^5$ einen gegebenenfalls ein-oder mehrfach durch Halogen substituierten $C_1$-$C_4$-Alkylrest,

$R^6$ einen gegebenenfalls ein-oder mehrfach durch Halogen substituierten $C_1$-$C_{12}$-Alkylrest oder einen gegebenenfalls ein-oder mehrfach, gleich oder verschieden durch $C_1$-$C_6$-Alkyl, Halogen, $C_1$-$C_4$-Alkoxy, Nitro und/oder Trifluormethyl substituierten Phenylrest und

n 1, 2, 3 oder 4

bedeuten.

2. Imidazo [2,1-a] isochinolin-2,5-6(3H)-trione der allgemeinen Formel I, in der $R^1$ Methyl, $R^2$ Methyl, Ethyl, Isopropyl und Cyclopropyl, $R^3$ Wasserstoff, $C_1$-$C_4$-Alkoxy, Allyloxy, Benzyloxy, Phenyloxy, und n 1 bedeuten.

3. Verfahren zur Herstellung von Verbindungen der allgemeinen Formel I, dadurch gekennzeichnet, daß man

a) Amide der allgemeinen Formel II

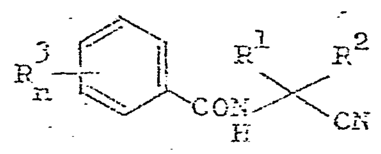

(II)

mit Oxalylchlorid umsetzt, oder

b) Imidazolinone der allgemeinen Formel III

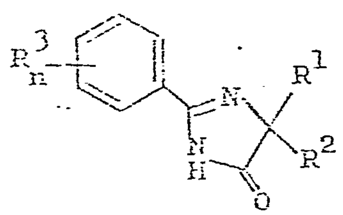

(III)

gegebenenfalls in Form der Hydrochloride mit Oxalylchlorid umsetzt, oder

c) Imidazolinone der allgemeinen Formel III

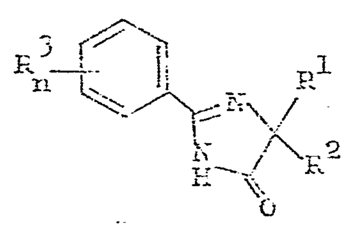

(III)

zunächst mit Alkyllithiumverbindungen der allgemeinen Formel IV

$R^7$ -Li (IV)

metalliert und anschließend mit Oxalsäureestern der allgemeinen Formel V

$R^8O_2C$ -$CO_2R^8$ (V)

umsetzt, wobei $R^1$, $R^2$, $R^3$ und n die oben angegebene Bedeutung haben und $R^7$ und $R^8$ jeweils für $C_1$-$C_4$-Alkyl steht.

4. Mittel mit herbizider Wirkung gekennzeichnet durch einen Gehalt an mindestens einer Verbindung gemäß den Ansprüchen 1 bis 2.

5. Mittel gemäß Anspruch 4 in Mischung mit Träger-und/oder Hilfsstoffen.

6. Mittel gemäß Anspruch 4 hergestellt nach Verfahren gemäß Anspruch 3.

7. Verwendung der Verbindungen gemäß den Ansprüchen 1 bis 2 zur Bekämpfung monokotyler und dikotyler Pflanzenarten in Nutzpflanzkulturen.

Patentansprüche (für den Vertragsstaat AT)

1. Herbizide Mittel, gekennzeichnet durch einen Gehalt an mindestens einem Imidazo [2,1-a] isochinolin-2,5,6(3H)-trion der allgemeinen Formel I

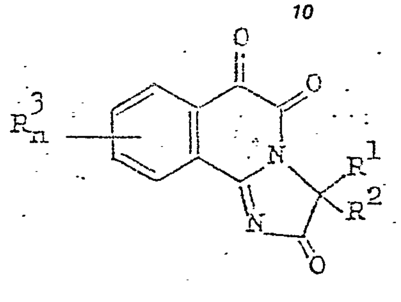

(I),

in der

$R^1$ einen gegebenenfalls ein-oder mehrfach durch Halogen substituierten $C_1$-$C_4$-Alkylrest,

$R^2$ einen gegebenenfalls ein-oder mehrfach durch Halogen substituierten $C_1$-$C_4$-Alkyl-oder $C_3$-$C_6$-Cycloalkylrest oder

$R^1$ und $R^2$ gemeinsam mit dem benachbarten Kohlenstoffatom einen gegebenenfalls durch Methyl substituierten $C_2$-$C_5$-Alkylenrest,

$R^3$ gleich oder verschieden Wasserstoff, Halogen, einen gegebenenfalls ein-oder mehrfach durch Halogen substituierten $C_1$-$C_4$-Alkylrest, einen gegebenenfalls ein-oder mehrfach, gleich oder verschieden durch $C_1$-$C_6$-Alkyl, Halogen, $C_1$-$C_4$-Alkoxy, $C_1$-$C_4$-Alkylthio, Nitro und/oder Trifluormethyl substituierten Phenylrest oder eine der Gruppen $R^4X$, $R^5_2N$ oder $R^6CONH$,

$R^4$ einen gegebenenfalls ein-oder mehrfach durch Halogen, Phenoxy und/oder $C_1$-$C_4$-Alkoxy substituierten $C_1$-$C_{12}$-Alkyl-, $C_2$-$C_{12}$-Alkenyl-oder $C_2$-$C_{12}$-Alkinylrest, einen gegebenenfalls ein-oder mehrfach durch Sauerstoff unterbrochenen $C_2$-$C_8$-Alkylrest, einen gegebenenfalls ein-oder mehrfach durch Halogen, $C_1$-$C_4$-Alkyl und/oder $C_1$-$C_4$-Alkoxy substituierten $C_3$-$C_6$-Cycloalkylrest, oder einen gegebenenfalls ein-oder mehrfach, gleich oder verschieden durch $C_1$-$C_6$-Alkyl, Halogen, $C_1$-$C_4$-Alkoxy, $C_1$-$C_4$-Alkylthio, Nitr und/oder Trifluormethyl substituierten Phenyl-oder Phenyl-$C_1$-$C_4$-Alkylrest,

X O oder S,

$R^5$ einen gegebenenfalls ein-oder mehrfach durch Halogen substituierten $C_1$-$C_4$-Alkylrest,

$R^6$ einen gegebenenfalls ein-oder mehrfach durch Halogen substituierten $C_1$-$C_{12}$-Alkylrest oder einen gegebenenfalls ein-oder mehrfach, gleich oder verschieden durch $C_1$-$C_6$-Alkyl, Halogen, $C_1$-$C_4$-Alkoxy, Nitro und/oder Trifluormethyl substituierten Phenylrest und

n 1, 2, 3 oder 4

bedeuten, als wirksamen Bestandteil.

2. Mittel gemäß Anspruch 1, gekennzeichnet durch einen Gehalt an mindestens einem Imidazo [2,1-a] isochinolin-2,5-6(3H)-trion der im Anspruch 1 angegebenen allgemeinen Formel I, in der $R^1$ Methyl, $R^2$ Methyl, Ethyl, Isopropyl und Cyclopropyl, $R^3$ Wasserstoff, $C_1$-$C_4$-Alkoxy, Allyloxy, Benzyloxy, Phenyloxy, und n 1 bedeuten, als wirksamen Bestandteil.

3. Verfahren zur Herstellung von Verbindungen der im Anspruch 1 angegebenen allgemeinen Formel I, dadurch gekennzeichnet, daß man

a) Amide der allgemeinen Formel II

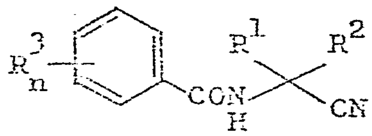

(II)

mit Oxalychlorid umsetzt, oder

b) Imidazolinone der allgemeinen Formel III

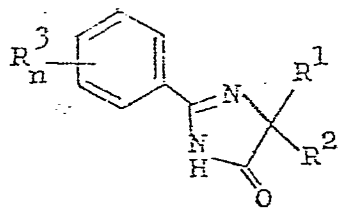

(III)

gegebenenfalls in Form der Hydrochloride mit Oxalylchlorid umsetzt, oder

c) Imidazolinone der allgemeinen Formel III

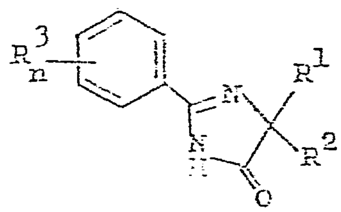

(III)

zunächst mit Alkyllithiumverbindungen der allgemeinen Formel IV

$R^7$ -Li (IV)

metalliert und anschließend mit Oxalsäureestern der allgemeinen Formel V

$R^8O_2C$ -$CO_2R^8$ (V)

umsetzt, wobei $R^1$, $R^2$, $R^3$ und n die oben angegebene Bedeutung haben und $R^7$ und $R^8$ jeweils für $C_1$-$C_4$-Alkyl steht.

4. Verwendung der Verbindungen gemäß den Ansprüchen 1 bis 2 zur Bekämpfung monokotyler und dikotyler Pflanzenarten in Nutzpflanzkulturen.